# FASCICULE DE BREVET EUROPEEN

(11) **EP 2 748 135 B1**
(45) Date de publication et mention de la délivrance du brevet: **17.06.2015**
(21) Numéro de dépôt: 12744112.9
(22) Date de dépôt: 26.07.2012
(51) Int. Cl.: C07C 22/04, C07C 253/22, C07C 229/08, C07C 255/07

(54) **PROCÉDÉ DE SYNTHÈSE D'ESTERS D'ACIDES OMEGA-AMINO-ALCANOIQUES EN C11 ET C12 COMPORTANT UNE ÉTAPE DE NITRILATON**
VERFAHREN ZUR HERSTELLUNG VON C11- UND C12-OMEGA-AMINOALKANSÄUREESTERN MIT EINEM NITRILIERUNGSSCHRITT
PROCESS FOR THE SYNTHESIS OF C11 AND C12 OMEGA-AMINOALKANOIC ACID ESTERS COMPRISING A NITRILATON STEP

(30) Priorité: 26.08.2011 FR 1157542
(43) Date de publication de la demande: 02.07.2014
(73) Titulaire: Arkema France, 92700 Colombes (FR)
(72) Inventeur: COUTURIER, Jean-Luc, 69006 Lyon (FR); DUBOIS, Jean-Luc, 69390 Millery (FR)
(74) Mandataire: Hérard, Elise
(86) Numéro de dépôt international: PCT/FR2012/051771
(87) Numéro de publication internationale: WO 2013/030481

(56) Documents cités:
- WO-A1-2010/055273
- JP-A- 4 208 260
- US-A- 6 005 134

## Description

Le travail qui a conduit à cette invention a reçu un financement de la part de l'Union Européenne dans le cadre du 7ième Programme Cadre (FP7/2007-2013) sous le numéro de projet N°241718 EUROBIOREF.

L'invention vise un procédé de synthèse d'esters d'acides ω-amino-alcanoïques en C11 et C12 comportant une étape de métathèse en utilisant comme matière première des esters d'acides ω-alcénoïques en C10 et C11.

L'industrie des polyamides utilise toute une gamme de monomères constitués par des ω-amino-acides à longue chaîne, usuellement appelés Nylon, caractérisés par la longueur de chaîne méthylène (-CH₂)ₙ séparant deux fonctions amide -CO-NH-, C'est ainsi que sont connus les nylon-6, nylon 6-6, nylon 6-10, nylon 7, nylon 8, nylon 9, nylon 11, nylon 13, etc...Les nylons « supérieurs » 11 et 13 par exemple utilisant comme monomère des ω-aminoacides occupent une place à part dans cette famille dans la mesure où ils ne sont pas synthétisés à partir des produits issus du pétrole (oléfines en C2 à C4, cycloalcanes ou benzène), mais à partir des acides/esters gras contenus dans les huiles naturelles.

Un exemple de procédé utilisant un acide gras comme matière première est celui de la fabrication de nitriles et/ou d'amines gras à partir d'acides gras extraits d'huiles végétales ou animales. Ce procédé est décrit dans l'encyclopédie Kirk-Othmer Vol 2, 4° Edition, page 411. L'amine grasse est obtenue en plusieurs étapes. La première étape consiste en une méthanolyse ou une hydrolyse d'une huile végétale ou d'une graisse animale produisant respectivement l'ester méthylique d'un acide gras ou un acide gras. L'ester méthylique de l'acide gras peut ensuite être hydrolyse pour former l'acide gras. Ensuite, l'acide gras est transformé en nitrile par réaction avec l'ammoniac, et finalement en amine par hydrogénation du nitrile ainsi obtenu.

L'évolution actuelle en matière d'environnement conduit d'ailleurs dans le domaine de la chimie à privilégier l'exploitation des matières premières naturelles provenant d'une source renouvelable. C'est la raison pour laquelle certains travaux de recherche-développement ont été repris pour élaborer sur le plan industriel des procédés utilisant des acides/esters gras comme matière première de fabrication de ces monomères ω-amino-acides.

Les principaux travaux ont porté sur la synthèse de l'acide amino-9-nonanoïque qui est le précurseur du Nylon 9 à partir de l'acide oléique d'origine naturelle. En ce qui concerne ce monomère on peut citer *l'ouvrage* « n-Nylons, Their Synthesis, Structure and Properties » - 1997 Ed.J. Wiley et Sons dont le chapitre 2.9 (pages 381 à 389) est consacré au 9-Nylon.

Sur le plan industriel pour la fabrication des monomères de polymérisation de type polyamide, il n'y a que peu d'exemples de procédés utilisant comme matière première des huiles naturelles. Un des rares exemples de procédé industriel utilisant un acide gras comme matière première est celui de la fabrication à partir de l'ester méthylique de l'acide ricinoléique, extrait de l'huile de ricin, de l'acide amino-11-undécanoïque, qui est à la base de la synthèse du Rilsan 11 ^{®}. Ce procédé est décrit dans l'ouvrage « Les Procédés de Pétrochimie de A. Chauvel et al. paru aux Editions TECHNIP (1986). L'acide amino-11-undécanoique est obtenu en plusieurs étapes. La première consiste en une méthanolyse de l'huile de ricin en milieu basique produisant le ricinoléate de méthyle qui est ensuite soumis à une pyrolyse pour obtenir, d'une part l'heptanaldéhyde et, d'autre part le undécylénate de méthyle. Ce dernier est passé en forme acide par hydrolyse. Ensuite l'acide formé est soumis à une hydrobromuration pour donner l'acide ω-bromé d'où l'on passe par amonolyse à l'acide amino-11-undécanoïque.

Depuis la demanderesse a poursuivi ses travaux sur les procédés de synthèse de ces monomères supérieurs fondés sur l'utilisation des huiles naturelles sources d'acides oléique, ricinoléique, lesquerolique, érucique ou autres. C'est ainsi qu'elle a exploré une voie dans laquelle la fonction acide était transformée en cours de processus en fonction nitrile par nitrilation (ammoniation) pour, après introduction dans la molécule d'une fonction acide ou ester par coupure oxydante ou métathèse avec un acrylate, être réduite en fonction amine primaire au terme des transformations. C'est dans ce cadre qu'elle a déposé une demande de brevet WO2010/055273 couvrant différentes variantes de mise oeuvre du procédé transitant toutes par la formation d'un nitrile gras ω-insaturé.

Dans le procédé de l'invention une étape importante est la nitrilation de la fonction acide/ester de l'acide gras insaturé.

Le schéma réactionnel de la synthèse des nitriles à partir d'un acide gras peut se résumer de la façon suivante.

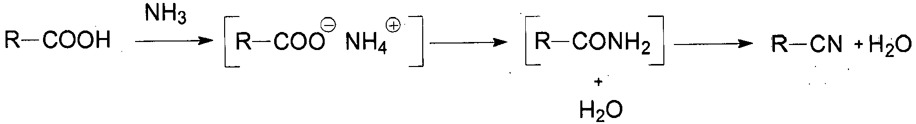

Il existe 2 types de procédés fondés sur ce schéma réactionnel : un procédé en phase liquide (en général en batch) et un procédé en phase vapeur (en général en continu).

Dans le procédé batch (phase liquide), on charge l'acide gras ou un mélange d'acides gras avec un catalyseur qui est généralement un oxyde métallique et le plus fréquemment l'oxyde de zinc. On porte le milieu réactionnel jusqu'à environ 150°C sous agitation, puis on commence à introduire l'ammoniac gazeux. Dans un premier temps, on forme un sel d'ammonium ou savon d'ammonium. La température du milieu réactionnel est ensuite portée aux alentours de 250°-300°C toujours sous introduction d'ammoniac. Le sel d'ammonium se transforme en amide avec libération d'une première molécule d'eau. Puis, dans un deuxième temps et avec l'aide du catalyseur, l'amide se transforme en nitrile avec formation d'une deuxième molécule d'eau. Cette eau formée est éliminée en continu du réacteur en entraînant l'ammoniac qui n'a pas réagi et un peu de chaînes grasses parmi les plus légères.

Dans le procédé en phase gaz (continu) la charge est vaporisée et amenée au contact avec de l'ammoniac dont la température est comprise entre 250 et 600 °C en présence d'un catalyseur. Ce catalyseur est en général choisi parmi la famille des oxydes métalliques constituée par les oxydes de métaux, pris seul ou en mélange, tels que Zr, Ta, Ga In, Sc, Nb, Hf, Fe, Zn, Sn ou de l'alumine, de la silice, un oxyde de thorium, et notamment de l'alumine dopée.

Ces réactions, sous leurs différentes formes sont mentionnées dans les Encyclopédies Ullmann vol. A2, page 20 et Kirk Othmer vol. 2 pages 411-412, et ont fait l'objet de nombreux brevets déposés notamment par la société KAO. On peut citer les brevets US 6,005,134, 6,080,891 et 7,259,274 qui décrivent la synthèse de nitriles aliphatiques en phase liquide à partir d'acides gras en présence d'un catalyseur au titane. Pour le même déposant et pour le même type de procédé, on peut relever les demandes japonaises N° 11-117990 (26/04/1999) avec un catalyseur au niobium et N° 9-4965 (14/01/1997) avec un catalyseur au zirconium. On peut également citer un brevet US n° 4,801,730 qui décrit la nitrilation des glycérides en phase liquide et une demande japonaise au nom de Lion Corp du 13/03/1991 (Publication N° JP 4283549) qui vise la synthèse de nitrile en phase gazeuse.

A l'occasion des travaux qu'elle a menés, la demanderesse a observé que l'étape de nitrilation jouait un rôle important notamment du fait qu'elle était réalisée sur un acide ω-insaturé. En effet la localisation de la double liaison en bout de chaîne et donc peu protégée, pouvait entraîner la formation d'isomères par suite du déplacement de la double liaison. Ayant observé ces phénomènes, elle a constaté que cet inconvénient pouvait être largement limité en travaillant avec l'ester plutôt que l'acide correspondant ce qui permettait d'opérer dans des conditions « plus douces ». En effet, le point d'ébullition de l'ester étant moins élevé que celui de l'acide correspondant il était possible d'atteindre des tensions de vapeur plus forte avec l'ester. En outre en opérant dans un réacteur fonctionnant en continu soit en phase gazeuse, soit en phase mixte liquide-gaz, le temps de séjour des réactifs au contact des catalyseurs étant nettement plus réduit qu'en phase liquide classique (batch) permettait de limiter l'isomérisation au cours du processus.

L'art antérieur décrit essentiellement la nitrilation en phase liquide de l'acide et ceux qui parlent de phase gaz ignorent le problème de l'isomérisation des doubles liaisons terminales, leurs objectifs étant fort différents de ceux du procédé de l'invention.

Le procédé de l'invention vise à pallier les inconvénients de l'art antérieur.

L'invention a pour objet un procédé de synthèse d'acides ou esters d'acides ω-amino-alcanoïques comportant 11 ou 12 atomes de carbone à partir d'acide ou d'ester ω-insaturé comportant respectivement 10 ou 11 atomes de carbone caractérisé en ce qu'il comporte trois étapes principales :
1) nitrilation de l'acide/ester ω-insaturé de la charge de formule CH₂=CH -(CH₂)ₙ-COOR dans laquelle n est 7 ou 8 et R soit H, soit un radical alkyle comportant 1 à 4 atomes de carbone, par action de l'ammoniac dans un réacteur fonctionnant en continu en phase gaz ou en phase mixte gaz-liquide en présence d'un catalyseur solide, puis
2) transformation du nitrile obtenu de formule CH₂=CH -(CH₂)ₙ-CN par métathèse avec un acrylate de formule CH₂=CH-COOR, où R₁ est soit H soit un radical alkyle comportant 1 à 4 atomes de carbone, et enfin
3) réduction par hydrogénation de la fonction nitrile du composé de formule R₁OOC-CH=CH -(CH₂)ₙ-CN conduisant à un amino-acide ou un amino-ester de formule R₁OOC-(CH₂)ₙ₊₂-CH₂NH₂.

L'étape de nitrilation est réalisée dans un réacteur fonctionnant en continu, c'est-à-dire dans lequel les réactifs qu'ils soient à l'origine gazeux ou liquide sont introduits (et les produits extraits) dans le réacteur en continu selon des débits prédéterminés.

Dans un premier mode de réalisation, les deux réactifs peuvent être introduits dans le réacteur à l'état gazeux (phase gazeuse pure).

Dans l'autre mode de réalisation (phase mixte) l'ammoniac est introduit sous forme de gaz alors que l'ester (acide) est introduit, après un préchauffage éventuel, dans le réacteur à proximité du lit catalytique, au moins en partie sous forme liquide à un débit déterminé pour s'écouler sous forme d'un film (lit ruisselant) sur le lit catalytique chauffé au contact duquel une fraction du liquide est vaporisée. La réaction ou la série de réactions s'effectue(nt) au contact de la surface du catalyseur ou dans sa proximité immédiate. Cette technique dit du « lit ruisselant » ést bien connue et largement employée dans l'industrie pétrolière. Le flux d'ammoniac peut être co-courant ou à contre-courant du flux d'ester

Le procédé de l'invention utilise comme charge des acides ou des esters ω-insaturés comportant soit 10 atomes, soit 11 atomes de carbone par molécule. Les premiers - notamment le 9-décénoate de méthyle - sont commercialisés sous forme d'ester par la société ELEVANCE Renewable Sciences, les seconds - notamment le 10-undécénoate de méthyle - sont fabriqués par la société ARKEMA dans le cadre de son procédé à base d'huile de ricin mentionné précédemment, l'undécylénate de méthyle étant obtenu après pyrolyse.

L'acrylate utilisé lors de la deuxième étape sera choisi parmi l'acide acrylique, l'acrylate de méthyle, l'acrylate d'éthyle, l'acrylate de n-propyle ou d'isopropyle, l'acrylate de n-*butyl*e, d'isobutyle, de sec-butyle ou de terbutyle.

Le schéma réactionnel du procédé est le suivant.

CH₂=CH -(CH₂)ₙ-COOR + NH₃ → CH₂=CH-(CH₂)ₙ-CN + ROH + H₂O

-CH₂=CH -(CH₂)ₙ-CN + CH₂=CH-COOR₁ → R₁OOC-CH=CH -(CH₂)ₙ-CN + CH₂=CH₂

R₁OOC-CH=CH-(CH₂)ₙ-CN + 3H₂ → R₁OOC-(CH₂)ₙ₊₂-CH₂NH₂.

### Etape Nitrilation

Le procédé de nitrilation catalytique des acides gras est conduit à une température de réaction généralement comprise entre 200 et 400 °C et de préférence entre 250 et 350 °C. La charge d'acides/esters gras ω-insaturés est vaporisée et amenée à une température comprise entre 180 et 350 °C au contact avec l'ammoniac dont la température d'introduction est comprise entre 150 et 600 °C.

La pression est comprise entre 10,13 et 1013 kPa (0,1 et 10 atmosphères) (absolues) et de préférence entre 50,65 et 506,5 kPa (0,5 et 5 atm) et de manière encore plus préférée entre 101,3 et 303,9 kPa (1 et 3 atm).

Lorsque l'étape de nitrilation est réalisée en phase gaz l'ester ω-insaturé d'acides gras est vaporisé et amené à une température comprise entre 180 et 350 °C au contact avec l'ammoniac dont la température d'introduction est comprise entre 150 et 600 °C et sous une pression comprise entre 10,13 et 1013 kPa (0,1 et 10 atmosphères) (absolues), de préférence entre 50,65 et 506,5 kPa (0,5 et 5 atm) et de manière encore plus préférée entre 101,3 et 303,9 kPa (1 et 3 atmosphères) et cela en présence du catalyseur solide, de préférence, la température de réaction est comprise entre entre 200°C et 400°C.

Dans la variante de réalisation du procédé totalement en phase gaz, les débits d'introduction des réactifs sont tels que le temps de contact avec le catalyseur solide est compris entre 1 seconde et 300 secondes. Dans ce cas le temps de contact est déterminé par le ratio calculé comme suit : {volume de catalyseur (en litres) x 3600} / {[débit de l'ester insaturé (en moles/h) + débit d'ammoniac (en moles/h)] x 22,4}=temps de contact en secondes.

Dans une variante du procédé, l'étape de nitrilation de l'ester ω-insaturé d'acides gras est réalisée en phase mixte selon la technique du lit ruisselant, l'ester ω-insaturé d'acides gras éventuellement préchauffé est envoyé progressivement sous forme liquide ruisselante sur le catalyseur solide chauffé à une température telle qu'il y ait vaporisation partielle progressive de l'ester permettant les réactions avec l'ammoniac au contact de la surface du catalyseur ou dans sa proximité immédiate. La température de réaction est généralement comprise entre 200 et 400 °C et de préférence entre 250 et 350 °C.Le débit d'introduction de l'ester est tel que le temps de séjour moyen de la phase liquide dans le réacteur est inférieur à 1 heure, et de préférence inférieur à 30 minutes. Ce temps de contact est déterminé par le calcul suivant : volume de catalyseur (en litres) / débit de l'ester insaturé (en litres liquides à 25 °C par heure), soit l'inverse de la vitesse volumique liquide horaire liquide.

Dans ce mode de mise en oeuvre, on peut travailler à co-courant, c'est-à-dire le courant gazeux et le flux liquide sont descendants, ou à contre-courant, le flux gazeux étant ascendant et le courant liquide descendant. Cette dernière variante est préférée dans le procédé de l'invention. La version contre-courant, gaz montant et ester descendant, peut être particulièrement intéressante pour limiter l'hydrolyse du nitrile formé. En effet dans cette configuration, l'ammoniac est injecté en pied, l'eau et l'alcool sortent en tête, l'ester entre en tête et le nitrile sort en pied. Donc en pied, on a une forte concentration en nitrile et en ammoniac et en tête une forte concentration en ester, eau et alcool, et moindre en ammoniac. On peut donc déplacer les équilibres, notamment celui de l'hydrolyse du nitrile qui redonne de l'acide.

Le ratio molaire NH₃/ester gras des réactifs est compris entre 1 et 50, de préférence entre 3 et 30, et de manière encore plus préférée compris entre 5 et 20.

La réaction est réalisée en présence d'un catalyseur solide.

Ce catalyseur est choisi parmi la famille des oxydes ou des oxydes mixtes métalliques constituée par des oxydes de métaux seuls ou en mélange, tels que Zr, Ce, Ti, Mo, W, V, S, P, Ta, Ga In, Sc, Nb, Hf, Fe, Zn, Sn, Al, Si. Les oxydes ou oxydes mixtes constituant le catalyseur peuvent être dopés par d'autres métaux dans le but d'augmenter les performances catalytiques. Les dopants qui conviennent à l'application incluent : les terres rares, La, Pr, Nd, Sm, Eu, Dy, Gd, Ho, Yb, ainsi que Cu, Ni, Fe, Pb, Sn, In, Mn, Co, Mo, W, Nb, Zn, Cr, Si, Mg, Ca, Sr, Sc, Y.

Les catalyseurs préférés dans le procédé de l'invention sont les oxydes à base de zirconium, de cérium, de titane, de niobium ou d'aluminium.

Avec l'oxyde de zirconium, on utilisera comme dopant les terres rares, avec une teneur de 5 à 50 % molaire, et de préférence de 8 à 15 %, mais aussi P, S, Cu, Ni, Fe, Pb, Sn, In, Mn, Mo, W, Nb, Zn, Cr, Si avec des teneurs de 1 à 30 % et de préférence supérieures à 5 %.

Avec l'oxyde de cérium on utilisera de préférence comme dopant : Mg, Ca, Sr, Sc, Y, et les terres rares, avec des teneurs de 1 à 50 %, et de préférence plus de 10%.

Avec l'oxyde de titane, on préférera comme dopant, W, Mo, P, S, Fe, Nb, Sn, Si, avec des teneurs de 1 à 50 % et de préférence de 5 à 20 %.

Comme méthode de préparation des catalyseurs, plusieurs méthodes peuvent convenir dont la coprécipitation, l'atomisation, le malaxage, l'imprégnation. Les précurseurs des oxydes sous différentes formes peuvent être utilisés notamment sous forme oxyde, nitrate, carbonate, chlorure, sulfate (y compris oxysulfate), phosphate, composé organométalique, acétate, acétylacétonate. Il est également possible d'utiliser les sels sous forme sulfate ou phosphate dans la préparation des catalyseurs dans la mesure où S ou P sont utilisés comme dopants du catalyseur. En l'occurrence la préparation d'un catalyseur à partir de l'oxysulfate de titane ou de zirconium conduit à un catalyseur convenable pour le procédé de l'invention.

Les catalyseurs ont une surface spécifique comprise entre 10 et 500 m²/g, et de préférence comprise entre 40 et 300 m²/g.

Les catalyseurs sont mis en forme selon les techniques qui conviennent suivant le type de réacteur utilisé.

Plusieurs technologies de réacteur peuvent convenir pour le procédé de l'invention : les réacteurs à lit fixe, les réacteurs à lit fluidisé en phase gaz.

Pour les réacteurs à lit fixe, les catalyseurs se présentent sous la forme de particules de 1 à 10 mm de granulométrie ou sous forme de monolithes poreux. Le catalyseur peut avoir alors différentes formes : billes, cylindres - creux ou non - bâtonnets...Le réacteur est utilisé soit en phase gaz uniquement ou encore en lit ruisselant où une phase gaz et une phase liquide co-existent.

Le réacteur peut être mis en oeuvre en lit fluidisé. Dans ce cas le catalyseur se présente sous la forme de poudre de 40 à 500 microns de diamètre et de préférence avec une granulométrie moyenne de 80 à 250 microns. Le débit de gaz de réactif NH₃ (gaz majoritaire dans le réacteur) est suffisant pour assurer la fluidisation du solide. Les esters et acides gras ayant des points d'ébullition élevés, il peut être avantageux d'injecter ces réactifs encore liquides directement dans le lit fluidisé de solide, le contact avec le catalyseur chaud assurant une vaporisation rapide des réactifs, ainsi qu'une augmentation du volume de gaz assurant la fluidisation. La régulation de la température du réacteur est assurée en partie par l'arrivée des réactifs liquides et gazeux à une température élevée et en partie par des épingles de circulation d'un fluide caloporteur installées au sein même du réacteur.

### Etape Métathèse

Les réactions de métathèse sont connues depuis longtemps même si leurs applications industrielles sont relativement limitées. On peut se référer à propos de leur utilisation dans la transformation des acides (esters) gras, à l'article de J.C. Mol « Catalytic metathesis of unsaturated fatty acid esters and oil » paru dans Topics in Catalysis Vol. 27, Nos. 1-4, February 2004, p 97 (Plenum Publishing).

La catalyse de la réaction de métathèse a fait l'objet de très nombreux travaux et le développement de systèmes catalytiques sophistiqués. On peut citer par exemple les complexes au tungstène développés par Schrock et al (J. Am. Chem. Soc. 108 (1986) 2771 ou Basset et al. Angew. Chem., Ed. Engl. 31 (1992) 628. Plus récemment, sont apparus les catalyseurs dits de Grubbs (Grubbs et al. Angew. Chem., Ed. Engl. 34 (1995) 2039 et Organic Lett. 1 (1999) 953) qui sont des complexes ruthénium-benzylidène. Il s'agit de catalyse homogène. Il a été aussi développé des catalyseurs hétérogènes à base de métaux tels que Rhénium, Molybdène et Tungstène déposés sur alumine ou silice.

Enfin des travaux ont été conduits pour la réalisation de catalyseurs immobilisés, c'est-à-dire de catalyseurs dont le principe actif est celui du catalyseur homogène, notamment les complexes ruthénium-carbène, mais qui est immobilisé sur un support inactif. L'objectif de ces travaux est d'augmenter la sélectivité de la réaction de métathèse croisée vis-à-vis des réactions parasites telles que les « homo-métathèses » entre les réactifs mis en présence. Ils portent non seulement sur la structure des catalyseurs mais également sur l'incidence du milieu réactionnel et les additifs pouvant être introduits. Ils portent également sur les méthodes de récupération du catalyseur après réaction.

Dans le procédé de l'invention, tout catalyseur de métathèse actif et sélectif pourra être utilisé. On utilisera cependant de préférence des catalyseurs à base de ruthénium.

La réaction de métathèse croisée avec le composé de type acrylate est réalisée dans des conditions parfaitement connues. La température de réaction est comprise entre 20 et 100 °C généralement à la pression atmosphérique, sous un flux de gaz inerte ou sous vide partiel, pour permettre un dégagement aisé de l'éthylène en présence d'un catalyseur à base de ruthénium.

Les catalyseurs au ruthénium sont choisis de préférence parmi les catalyseurs chargés ou non-chargés de formule générale :

(X1)a (X2)bRu(carbène C) (L1)c(L2)d

dans laquelle :
- a, b, c, d sont des nombres entiers avec a et b égaux à 0, 1 ou 2 ; c et d égaux à 0, 1, 2, 3 ou 4 ;
- X1 et X2, identiques ou différents, représentent chacun un ligand mono- ou multi-chélatant, chargé ou non ; à titre d'exemples, on pourra citer les halogénures, le sulfate, le carbonate, les carboxylates, les alcoolates, les phénolates, les amidures, le tosylate, l'hexafluorophosphate, le tétrafluoroborate, le bis-triflylamidure, le tétraphénylborate et dérivés.
- X1 ou X2 peuvent être liés à L1 ou L2 (Y1 ou Y2) ou au (carbène C) de façon à former un ligand bidenté (ou chélate) sur le ruthénium ; et
- L1 et L2 identiques ou différents, sont des ligands donneurs d'électrons tels que phosphine, phosphite, phosphonite, phosphinite, arsine, stilbine, une oléfine ou un aromatique, un composé carbonylé, un éther, un alcool, une amine, une pyridine ou dérivé, une imine, un thioéthers, ou un carbène hétérocyclique
- L1 ou L2 peuvent être liés au (carbène C) de façon à former un ligand bidenté ou chélate,

Le (carbène C) pourra être représenté par la formule générale : C_(R1)_(R2) pour laquelle R1 et R2 sont identiques ou différents tels que l'hydrogène ou tout autre groupe hydrocarbonyle saturé ou insaturé, cyclique, branché ou linéaire, ou aromatique. A titre d'exemples, on pourra citer les complexes du ruthénium alkylidènes, ou cumulènes tels que les vinylidènes Ru=C=CHRou allénylidènes Ru=C=C=CR1 R2 ou indénylidènes.

Un groupe fonctionnel permettant d'améliorer la rétention du complexe du ruthénium dans le liquide ionique peut être greffé sur au moins l'un des ligands X1, X2, L1, L2, ou sur le carbène C. Ce groupe fonctionnel peut être chargé ou non chargé tel que de préférence un ester, un éther, un thiol, un acide, un alcool, une amine, un hétérocycle azoté, un sulfonate, un carboxylate, un ammonium quaternaire, un guanidinium, un phosphonium quaternaire, un pyridinium, un imidazolium, un morpholinium ou un sulfonium.

### Etape Hydrogénation

L'étape de synthèse des ω-amino-esters ou ω-amino-acides gras à partir des nitrile-esters (acides) gras insaturés consiste en une hydrogénation classique mentionnée dans les Encyclopédies mentionnées précédemment aux mêmes chapitres. L'hydrogénation de la fonction nitrile entraîne automatiquement la saturation de la double liaison présente dans la molécule.

La réduction de la fonction nitrile en amine primaire est bien connue de l'homme de l'art. L'hydrogénation peut être conduite en présence de métaux précieux (Pt, Pd, Rh, Ru...) à une température comprise entre 20 et 100°C sous une pression de 1 à 5 bars. Elle peut également être conduite en présence de catalyseurs à base de fer, nickel ou cobalt, qui peuvent entraîner des conditions plus sévères avec des températures de l'ordre de 150 °C et des pressions élevées de plusieurs dizaines de bar. Les catalyseurs sont nombreux mais préférentiellement on utilise les Nickel et Cobalt de Raney. Afin de favoriser la formation de l'amine primaire, on opère avec une pression partielle d'ammoniac.

De préférence, l'étape de réduction des nitrile-esters (acides) gras en ω-amino-esters ou ω-amino-acides gras consiste en une hydrogénation utilisant tout catalyseur classique et de préférence les Nickel et Cobalt de Raney.

De préférence, la charge traitée est sous forme d'ester d'acide gras ω-insaturé.

Le procédé de l'invention est illustré par les exemples suivants donnés à titre non limitatif.

### Exemple1 : Nitrilation

On utilise comme élément actif du catalyseur un oxyde de titane de type Anatase ST 31119 produit par la société Saint-Gobain, ayant une surface spécifique de 48 m²/g. L'oxyde de titane est imprégné par une solution de paratungstate d'ammonium de manière à obtenir un dépôt homogène d'oxyde de tungstène de 5 % poids. Le solide est ensuite calciné sous flux d'air à 400 °C pendant 2 heures.

1 g de catalyseur est placé dans un réacteur tubulaire de 10 mm de diamètre. Au dessus du lit catalytique on place du carbure de silicium qui assure un préchauffage des réactifs. Le réacteur est alimenté avec un mélange gazeux d'ammoniac et d'undécylénate de méthyle, dans un ratio molaire de 5/1 et avec une VVH de 600 h⁻¹, soit un temps de contact d'environ 5 secondes. Les réactifs sont préchauffés très rapidement à 250 °C avant d'arriver dans le réacteur. Après réaction les gaz sont refroidis à environ 120 °C, pour permettre la condensation du nitrile, et des réactifs non convertis, et pour maintenir l'ammoniac ainsi que l'eau et le méthanol produits en phase gaz. Les produits de la réaction sont ensuite analysés par chromatographie.

La conversion de l'undécylénate de méthyle est de 99,5 %, le rendement en nitrile est de 96 %, à une température de réaction de 300 °C. La sélectivité en 10-cyanodécène (nitrile omega insaturé) est de 95 %, par rapport à la totalité des nitriles produits.

### Exemple 2 (comparatif)

Cet exemple illustre l'étape de nitrilation en phase liquide classique transformant de l'acide 10-undécénoïque en nitrile de formule CN-(CH₂)₈- CH=CH₂.

La réaction de nitrilation de l'acide 10-undécénoïque (3,5 g) pour former le nitrile ω-insaturé de formule CN-(CH₂)₈-CH=CH₂ est conduite en batch. Le milieu réactionnel est porté à 160 °C à une vitesse de 1°C par minute. L'introduction d'ammoniac (0,417 litre/kg acide.mn) commence lorsque les 160 °C sont atteints en palier, et on maintient cette température jusqu'à ce que l'indice d'acide du milieu tombe en dessous de 0,1 mg KOH/g. La température est ensuite augmentée jusqu'à 265 °C (la température est limitée par la forte évaporation du milieu à la pression de travail). La réaction est arrêtée après 18 heures. Pendant toute la synthèse un déflegmateur localisé en aval du réacteur est maintenu à 130 °C. La réaction est effectuée à la pression atmosphérique en présence d'un catalyseur d'oxyde de zinc (0,0625 % poids par rapport à l'acide). L'élimination en continu de l'eau formée entraîne l'ammoniac en excès et permet un achèvement rapide de la réaction. On récupère 2,6 g du nitrile qui sont séparés par distillation sous vide. Le nitrile oméga insaturé représente 90 % des nitriles obtenus.

### Exemple 3 : Métathèse croisée

Cet exemple illustre la réaction de métathèse croisée de l'undécénenitrile de formule CN-(CH₂)₈- CH=CH₂ avec l'acrylate de méthyle avec le catalyseur de Hoveyda-Grubbs Il :

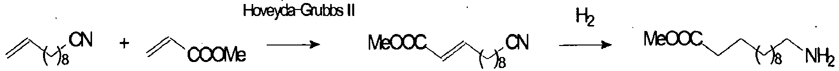

Dans un tube de Schlenk de 50 ml purgé à l'azote, on charge 83 mg de 10-undécènenitrile (0,5 mmol), 86 mg d'acrylate de méthyle (1 mmol) et 10 ml de toluène distillé sur sodium benzophénone. On ajoute 9,5 mg de catalyseur de Hoveyda-Grubbs de 2^{nd} génération (1,5.10⁻² mmol) et on chauffe à 100 °C pendant 1 heure.

L'analyse par chromatographie en phase gaz montre que la conversion du 10-undécènitrile est de 100% et que le rendement en nitrile-ester insaturé est de 98%.

### Exemple 4 : Hydrogénation

Le mélange réactionnel issu de l'exemple 3 est alors transféré dans une bombe de Parr de 50 ml (remplie à 22 ml). On ajoute 10 mg de catalyseur 1 % Pd/C et 17 mg de tertiobutylate de potassium (0,15 mmol) et on pressurise sous 20 bars d'hydrogène. Sous agitation magnétique, on chauffe à 80 °C pendant 48 h.

L'analyse par chromatographie en phase gaz montre que la conversion du nitrile-ester insaturé est de 90 % et que le rendement en 12-amino-dodécanoate de méthyle est de 64 %.

### Exemple 5 (comparatif)

Cet exemple illustre l'étape de nitrilation en phase liquide classique transformant de l'ester méthylique de l'acide 10-undécénoïqué en nitrile de formule CN-(CH₂)₈- CH=CH₂.

La réaction de nitrilation de l'ester de l'acide 10-undécénoïque (3,77 g) pour former le nitrile ω-insaturé de formule CN-(CH₂)₈-CH=CH₂ est conduite en batch, et en phase liquide. On procède comme dans le cas de l'exemple 2. Le milieu réactionnel est porté à 160 °C à une vitesse de 1°C par minute. L'introduction d'ammoniac (0,417 litre/kg acide.mn) commence lorsque les 160 °C sont atteints en palier. L'indice d'acide reste faible puisqu'on est en présence d'un ester. La température est ensuite augmentée jusqu'à 240 °C (la température est limitée par le point d'ébullition de l'ester méthylique). La réaction est effectuée avec un reflux total de l'ester méthylique et est de ce fait très difficile à conduire, et énergivore. La température de réaction augmente progressivement avec la conversion de l'ester méthylique, pour être plafonnée par l'ébullition du produit recherché : le 10-undécènenitrile. La réaction est arrêtée après 18 heures. Pendant toute la synthèse un déflegmateur localisé en aval du réacteur est maintenu à 130 °C. La réaction est effectuée à la pression atmosphérique en présence d'un catalyseur d'oxyde de zinc (0,0625 % poids par rapport à l'acide). L'élimination en continu de l'eau formée entraîne l'ammoniac en excès et permet un achèvement rapide de la réaction. On récupère 1,2 g du nitrile qui sont séparés par distillation sous vide. Le nitrile oméga insaturé représente 85 % des nitriles obtenus.

### Exemple 6 : Nitrilation

Nb₂O₅ fraichement préparé par hydrolyse de chlorure de niobium, (jusqu'à absence de chlorure dans les eaux de lavage au test au nitrate d'argent), puis calcination à 300 °C sous air pendant 1 heure, est utilisé comme catalyseur.

5 g de catalyseur sont placés dans un réacteur tubulaire de 10 mm de diamètre. Au dessus du lit catalytique on place du carbure de silicium qui assure un préchauffage des réactifs. Le réacteur est alimenté avec un mélange gazeux d'ammoniac et d'undécylénate de méthyle, dans un ratio molaire de 5/1 et avec une VVH de 120 h⁻¹, soit un temps de contact d'environ 30 secondes. Les réactifs sont préchauffés très rapidement à 200 °C avant d'arriver dans le réacteur. Après réaction les gaz sont refroidis à environ 120 °C, pour permettre la condensation du nitrile, et des réactifs non convertis, et pour maintenir l'ammoniac, ainsi que l'eau et le méthanol produits en phase gaz. Les produits de la réaction sont ensuite analysés par chromatographie.

La conversion de l'undécylénate de méthyle est de 97 %, le rendement en nitrile est de 95 %, à une température de réaction de 250 °C. La sélectivité en 10-cyanodécène ou 10-undécènenitrile (nitrile omega insaturé) est de 96 %, par rapport à la totalité des nitriles produits.

## Revendications

1. Procédé de synthèse d'acides ou esters d'acides ω-amino-alcanoïques comportant 11 ou 12 atomes de carbone à partir d'acide ou d'ester ω-insaturé comportant respectivement 10 ou 11 atomes de carbone **caractérisé en ce qu'**il comporte trois étapes principales :
- nitrilation de l'acide/estér ω-insaturé de la charge de formule CH₂=CH -(CH₂)ₙ-COOR dans laquelle n est 7 ou 8 et R soit H, soit un radical alkyle comportant 1 à 4 atomes de carbone, par action de l'ammoniac dans un réacteur fonctionnant en continu en phase gaz ou en phase mixte gaz-liquide en présence d'un catalyseur solide, puis
- transformation du nitrile obtenu de formule CH₂=CH -(CH₂)ₙ-CN par métathèse avec un acrylate de formule CH₂=CH-COOR₁ où R₁ est soit H, soit un radical alkyle comportant 1 à 4 atomes de carbone et enfin
- réduction par hydrogénation de la fonction nitrile du composé de formule R₁OOC-CH=CH -(CH₂)ₙ-CN conduisant à un amino-acide ou un amino-ester de formule R₁OOC-(CH₂)ₙ₊₂-CH₂NH₂.

2. Procédé selon la revendication 1 **caractérisé en ce que** lors de l'étape de nitrilation réalisée en phase gaz l'ester ω-insaturé d'acides gras est vaporisé et amené à une température comprise entre 180 et 350 °C au contact avec l'ammoniac dont la température d'introduction est comprise entre 150 et 600 °C et sous une pression comprise entre 10,13 et 1013 kPa (0,1 et 10 atmosphères) (absolues), de préférence entre 50,65 et 506,5 kPa (0,5 et 5 atm) et de manière encore plus préférée entre 101,3 et 303,9 kPa (1 et 3 atmosphères) et cela en présence du catalyseur solide.

3. Procédé selon la revendication 2 **caractérisé en ce que** la température de réaction est généralement comprise entre 200 et 400 °C et de préférence entre 250 et 350 °C, et que les débits d'introduction des réactifs sont tels que le temps de contact avec le catalyseur solide est compris entre 1 seconde et 300 secondes.

4. Procédé selon la revendication 1 **caractérisé en ce que** l'étape de nitrilation de l'ester ω-insaturé d'acides gras est réalisée en phase mixte selon la technique du lit ruisselant, l'ester. ω-insaturé d'acides gras, éventuellement préchauffé, est envoyé progressivement sous forme liquide ruisselante sur le catalyseur solide chauffé à une température telle qu'il y ait vaporisation partielle progressive de l'ester permettant les réactions avec l'ammoniac au contact de la surface du catalyseur ou dans sa proximité immédiate.

5. Procédé selon la revendication 4 **caractérisé en ce que** la température de réaction est généralement comprise entre 200 et 400 °C et de préférence entre 250 et 350 °C, et que le débit d'introduction de l'ester est tel que le temps de séjour moyen de la phase liquide dans le réacteur est inférieur à 1 heure, et de préférence inférieur à 30 minutes.

6. Procédé selon l'une des revendications 1 à 5 **caractérisé en ce que** le ratio molaire NH₃/ester gras des réactifs est compris entre 1 et 50, de préférence entre 3 et 30, et de manière encore plus préférée compris entre 5 et 20.

7. Procédé selon l'une des revendications 1 à 6 **caractérisé en ce que** le catalyseur solide est choisi parmi la famille des oxydes métalliques constituée par les oxydes de métaux, seuls ou en mélange, tels que Zr, Ce, Ti, Mo, W, V, S, P, Ta, Ga, In, Sc, Nb, Hf, Fe, Zn, Sn, Al, Si.

8. Procédé selon la revendication 7 **caractérisé en ce que** les oxydes ou oxydes mixtes constituant le catalyseur peuvent être dopés par d'autres métaux, les dopants convenant à l'application incluent : les terres rares, La, Pr, Nd, Sm, Eu, Dy, Gd, Ho, Yb, ainsi que Cu, Ni, Fe, Pb, Sn, In, Mn, Co, Mo, W, Nb, Zn, Cr, Si, Mg, Ca, Sr, Sc, et Y.

9. Procédé selon l'une des revendications 7 et 8 **caractérisé en ce que** les catalyseurs ont une surface spécifique comprise entre 10 et 500 m²/g, et de préférence comprise entre 40 et 300 m²/g.

10. Procédé selon l'une des revendications 1 à 9 **caractérisé en ce que** la réaction de métathèse croisée avec le composé de type acrylate est réalisée à une température comprise entre 20 et 100 °C généralement à la pression atmosphérique pour permettre un dégagement aisé de l'éthylène en présence d'un catalyseur à base de ruthénium.

11. Procédé selon la revendication 10 **caractérisé en ce que** les catalyseurs au ruthénium sont choisis de préférence parmi les catalyseurs chargés ou non-chargés de formule générale :
(X1)a (X2)bRu(carbène C) (L1)c(L2)d
dans laquelle :
• a, b, c, d sont des nombres entiers avec a et b égaux à 0, 1 ou 2 ; c et d égaux à 0, 1, 2, 3 ou 4,
• X1 et X2, identiques ou différents, représentent chacun un ligand mono- ou multi-chélatant, chargé ou non ;
• X1 ou X2 peuvent être liés à L1 ou L2 ou au (carbène C) de façon à former un ligand bidenté (ou chélate) sur le ruthénium, et
• L1 et L2 identiques ou différents, sont des ligands donneurs d'électrons tels que phosphine, phosphite, phosphonite, phosphinite, arsine, stilbine, une oléfine ou un aromatique, un composé carbonylé, un éther, un alcool, une amine, une pyridine ou dérivé, une imine, un thioéthers, ou un carbène hétérocyclique
• L1 ou L2 pouvant être liés au (carbène C) de façon à former un ligand bidenté ou chélate,
• le (carbène C) pouvant être représenté par la formule générale : C_(R1)_(R2) pour laquelle R1 et R2 sont identiques ou différents tels que l'hydrogène ou tout autre groupe hydrocarbonyle saturé ou insaturé, cyclique, branché ou linéaire, ou aromatique.

12. Procédé selon l'une des revendications 1 à 11 **caractérisé en ce que** l'étape de réduction des nitrile-esters (acides) gras en ω-amino-esters ou ω-amino-acides gras consiste en une hydrogénation utilisant tout catalyseur classique et de préférence les Nickel et Cobalt de Raney.

13. Procédé selon l'une des revendications 1 à 12 **caractérisé en ce que** la charge traitée est sous forme d'ester d'acide gras ω-insaturé.

## Patentansprüche

1. Verfahren zur Synthese von Säuren oder Estern von ω-Aminoalkansäuren mit 11 oder 12 Kohlenstoffatomen aus ω-ungesättigter Säure bzw. ω-ungesättigtem Ester mit 10 bzw. 11 Kohlenstoffatomen, **gekennzeichnet durch** die folgenden drei Hauptschritte:
- Nitrilierung der ω-ungesättigten Säure/des ω-ungesättigen Esters der Charge der Formel CH₂=CH-(CH₂)ₙ-COOR, worin n für 7 oder 8 steht und R entweder für H oder für einen Alkylrest mit 1 bis 4 Kohlenstoffatomen steht, **durch** Einwirkung von Ammoniak in einem kontinuierlich betriebenen Reaktor in der Gasphase oder in der Gas-Flüssigkeits-Mischphase in Gegenwart eines festen Katalysators, dann
- Umwandlung des erhaltenen Nitrils der Formel CH₂=CH-(CH₂)ₙ-CN **durch** Metathese mit einem Acrylat der Formel CH₂=CH-COOR₁, wobei R₁ entweder für H oder für einen Alkylrest mit 1 bis 4 Kohlenstoffatomen steht, und schließlich
- Reduktion der Nitrilfunktion der Verbindung der Formel R₁OOC-CH=CH-(CH₂)ₙ-CN **durch** Hydrierung, was zu einer Aminosäure bzw. einem Aminoester der Formel R₁OOC-(CH₂)ₙ₊₂-CH₂NH₂ führt.

2. Verfahren nach Anspruch 1, **dadurch gekennzeichnet, dass** in dem in der Gasphase durchgeführten Nitrilierungsschritt der ω-ungesättigte Fettsäureester verdampft und bei einer Temperatur zwischen 180 und 350°C und unter einem Druck zwischen 10,13 und 1013 kPa (0,1 und 10 Atmosphären) (absolut), vorzugsweise zwischen 50,65 und 506,5 kPa (0,5 und 5 Atmosphären) und noch weiter bevorzugt zwischen 101,3 und 303,9 kPa (1 und 3 Atmosphären) in Gegenwart des festen Katalysators mit Ammoniak, dessen Eintragungstemperatur zwischen 150 und 600°C liegt, in Kontakt gebracht wird.

3. Verfahren nach Anspruch 2, **dadurch gekennzeichnet, dass** die Reaktionstemperatur im Allgemeinen zwischen 200 und 400°C und vorzugsweise zwischen 250 und 350°C liegt und dass die Eintragsraten der Reaktanten so beschaffen sind, dass die Kontaktzeit mit dem festen Katalysator zwischen 1 Sekunde und 300 Sekunden liegt.

4. Verfahren nach Anspruch 1, **dadurch gekennzeichnet, dass** der Schritt der Nitrilierung des ω-ungesättigten Fettsäureesters in der Mischphase gemäß der Rieselbett-Technik durchgeführt wird, der gegebenenfalls vorerhitzte ω-ungesättigte Fettsäureester allmählich in rieselnder flüssiger Form auf den festen Katalysator gegeben wird, welcher auf eine solche Temperatur erhitzt wird, dass der Ester allmählich teilweise verdampft, so dass die Reaktionen mit Ammoniak in Kontakt mit der Oberfläche des Katalysators oder in unmittelbarer Nähe davon ablaufen können.

5. Verfahren nach Anspruch 4, **dadurch gekennzeichnet, dass** die Reaktionstemperatur im Allgemeinen zwischen 200 und 400°C und vorzugsweise zwischen 250 und 350°C liegt und dass die Eintragsrate des Esters so beschaffen ist, dass die mittlere Verweilzeit der flüssigen Phase im Reaktor weniger als 1 Stunde und vorzugsweise weniger als 30 Minuten beträgt.

6. Verfahren nach einem der Ansprüche 1 bis 5, **dadurch gekennzeichnet, dass** das NH₃/Fettester-Molverhältnis der Reaktanten zwischen 1 und 50, vorzugsweise zwischen 3 und 30 und noch weiter bevorzugt zwischen 5 und 20 liegt.

7. Verfahren nach einem der Ansprüche 1 bis 6, **dadurch gekennzeichnet, dass** der feste Katalysator aus der Klasse der Metalloxide bestehend aus den Oxiden von Metallen, allein oder als Mischung, wie Zr, Ce, Ti, Mo, W, V, S, P, Ta, Ga, In, Sc, Nb, Hf, Fe, Zn, Sn, Al, Si, ausgewählt wird.

8. Verfahren nach Anspruch 7, **dadurch gekennzeichnet, dass** die Oxide oder gemischten Oxide, aus denen der Katalysator besteht, mit anderen Metallen dotiert sein können, wobei die zur Anwendung geeigneten Dotierstoffe die folgenden einschließen: seltene Erden, La, Pr, Nd, Sm, Eu, Dy, Gd, Ho, Yb, sowie Cu, Ni, Fe, Pb, Sn, In, Mn, Co, Mo, W, Nb, Zn, Cr, Si, Mg, Ca, Sr, Sc und Y.

9. Verfahren nach einem der Ansprüche 7 und 8, **dadurch gekennzeichnet, dass** die Katalysatoren eine spezifische Oberfläche zwischen 10 und 500 m²/g und vorzugsweise zwischen 40 und 300 m²/g aufweisen.

10. Verfahren nach einem der Ansprüche 1 bis 9, **dadurch gekennzeichnet, dass** die Kreuzmetathesereaktion mit der Verbindung vom Acrylat-Typ bei einer Temperatur zwischen 20 und 100°C, im Allgemeinen bei Normaldruck, um die Freisetzung von Ethylen zu erlauben, in Gegenwart eines Rutheniumkatalysators durchgeführt wird.

11. Verfahren nach Anspruch 10, **dadurch gekennzeichnet, dass** die Rutheniumkatalysatoren vorzugsweise aus geladenen oder ungeladenen Katalysatoren der folgenden allgemeinen Formel ausgewählt werden:
(X1)a(X2)bRu(Carben C)(L1)c(L2)d
wobei:
• a, b, c und d ganze Zahlen sind, wobei a und b gleich 0, 1 oder 2 sind und c und d gleich 0, 1, 2, 3 oder 4 sind,
• X1 und X2 gleich oder verschieden sind und jeweils für einen geladenen oder ungeladenen Mono- oder Multichelatliganden stehen,
• X1 und X2 an L1 oder L2 oder (Carben C) gebunden sein können, so dass sich ein zweizähniger Ligand (oder Chelatligand) am Ruthenium ergibt, und
• L1 und L2 gleich oder verschieden sind und für elektronenliefernde Liganden wie Phosphit, Phosphit, Phosphonit, Phosphinit, Arsin, Stilbin, ein Olefin oder einen Aromaten, eine Carbonylverbindung, einen Ether, einen Alkohol, ein Amin, ein Pyridin oder Pyridinderivat, ein Imin, einen Thioether oder ein heterocyclisches Carben stehen,
• wobei L1 und L2 an (Carben C) gebunden sein können, so dass sich ein zweizähniger Ligand oder Chelatligand ergibt,
• wobei das (Carben C) durch die folgende allgemeine Formel wiedergegeben werden kann: C_(R1)_(R2), wobei R1 und R2 gleich oder verschieden sind, wie Wasserstoff oder eine beliebige andere gesättigte oder ungesättige cyclische, verzweigte oder lineare oder aromatische Kohlenwasserstoffgruppe.

12. Verfahren nach einem der Ansprüche 1 bis 11, **dadurch gekennzeichnet, dass** der Schritt der Reduktion der Nitrilfettester(säuren) zu ω-Aminoestern bzw. ω-Aminofettsäuren aus einer Hydrierung unter Verwendung eines beliebigen herkömmlichen Katalysators, vorzugsweise Raney-Nickel oder -Cobalt, besteht.

13. Verfahren nach einem der Ansprüche 1 bis 12, **dadurch gekennzeichnet, dass** die behandelte Charge in Form von ω-ungesättigtem Fettsäureester vorliegt.

## Claims

1. A process for synthesizing acids or esters of ω-aminoalkanoic acids comprising 11 or 12 carbon atoms from ω-unsaturated acid or ester comprising respectively 10 or 11 carbon atoms, **characterized in that** it comprises three main steps:
- nitrilation of the ω-unsaturated acid/ester of the charge, of formula CH₂-CH-(CH₂)ₙ-COOR, in which n is 7 or 8 and R is either H or an alkyl radical comprising 1 to 4 carbon atoms, by action of ammonia, in a reactor operating continuously in gas phase or in mixed gas-liquid phase, in the presence of a solid catalyst, then
- conversion of the resulting nitrile of formula CH₂-CH-(CH₂)ₙ-CN by metathesis with an acrylate of formula CH₂-CH-COOR₁, where R₁ is either H or an alkyl radical comprising 1 to 4 carbon atoms, and lastly
- hydrogenative reduction of the nitrile function of the compound of formula R₁OOC-CH=CH-(CH₂)ₙ-CN to give an amino acid or an amino ester of formula R₁OOC-(CH₂)ₙ₊₂ - CH₂NH₂.

2. The process as claimed in claim 1, **characterized in that** in the nitrilation step performed in gas phase, the ω-unsaturated fatty acid ester is evaporated and brought at a temperature of between 180 and 350°C into contact with ammonia introduced at a temperature of between 150 and 600°C and under a pressure of between 10.13 and 1013 KPa (0.1 and 10 atmospheres) (absolute), preferably between 50.65 and 506,5 KPa (0.5 and 5 atmospheres), and more preferably between 101.3 and 303.9 KPa (1 and 3 atmospheres), in the presence of the solid catalyst.

3. The process as claimed in claim 2, **characterized in that** the reaction temperature is generally between 200 and 400°C and preferably between 250 and 350°C, and **in that** the rates at which the reactants are introduced are such that the contact time with the solid catalyst is between 1 second and 300 seconds.

4. The process as claimed in claim 1, **characterized in that** the step of nitrilation of the ω-unsaturated fatty acid ester is carried out in mixed phase according to the trickle bed technique, with the optionally preheated ω-unsaturated fatty acid ester being conveyed progressively in trickling liquid form over the solid catalyst heated at a temperature such that there is partial, progressive evaporation of the ester, permitting the reactions with ammonia on contact with the surface of the catalyst or in its immediate proximity.

5. The process as claimed in claim 4, **characterized in that** the reaction temperature is generally between 200 and 400°C and preferably between 250 and 350°C, and **in that** the rate at which the ester is introduced is such that the mean residence time of the liquid phase in the reactor is less than 1 hour, and preferably less than 30 minutes.

6. The process as claimed in any of claims 1 to 5, **characterized in that** the molar NH₃/fatty ester ratio of the reactants is between 1 and 50, preferably between 3 and 30, and more preferably between 5 and 20.

7. The process as claimed in any of claims 1 to 6, **characterized in that** the solid catalyst is selected from the class of metal oxides consisting of the oxides of metals, alone or in a mixture, such as Zr, Ce, Ti, Mo, W, V, S, P, Ta, Ga, In, Sc, Nb, Hf, Fe, Zn, Sn, Al, Si.

8. The process as claimed in claim 7, **characterized in that** the oxides or mixed oxides that constitute the catalyst may be doped with other metals, the dopants suitable for application including the following: rare earths, La, Pr, Nd, Sm, Eu, Dy, Gd, Ho, Yb, and also Cu, Ni, Fe, Pb, Sn, In, Mn, Co, Mo, W, Nb, Zn, Cr, Si, Mg, Ca, Sr, Sc, and Y.

9. The process as claimed in either of claims 7 and 8, **characterized in that** the catalysts have a specific surface area between 10 and 500 m²/g, and preferably of between 40 and 300 m²/g_{.}

10. The process as claimed in any of claims 1 to 9, **characterized in that** the cross metathesis reaction with the acrylate compound is carried out at a temperature of between 20 and 100°C., generally at atmospheric pressure, to allow easy release of ethylene, in the presence of a ruthenium-based catalyst.

11. The process as claimed in claim 10, **characterized in that** the ruthenium catalysts are selected preferably from charged or noncharged catalysts of general formula:
(X1)a (X2)bRu(carbene C) (L1)c(L2)d
in which:
• a, b, c, and d are integers, with a and b being 0, 1 or 2; c and d being 0, 1, 2, 3, or 4,
• X1 and X2, which are identical or different, each represent a charged or noncharged unidentate or multidentate ligand,
• X1 or X2 may be bonded to L1 or L2 or to the (carbene C) so as to form a bidentate (or chelate) ligand on the ruthenium, and
• L1 and L2, which are identical or different, are electron-donating ligands such as phosphine, phosphite, phosphonite, phosphinite, arsine, stilbine, an olefin or an aromatic, a carbonyl compound, an ether, an alcohol, an amine, a pyridine or derivative, an imine, a thioether, or a heterocyclic carbene,
• L1 or L2 may be bonded to the (carbene C) so as to form a bidentate or chelate ligand,
• the (carbene C) may be represented by the general formula: C_(R1)_(R2), for which R1 and R2 are identical or different, such as hydrogen or any other saturated or unsaturated cyclic, branched, or linear hydrocarbon group, or aromatic hydrocarbon group.

12. The process as claimed in any of claims 1 to 11, **characterized in that** the step of reduction of the fatty (acid) nitrile esters to ω-amino esters or ω-amino-fatty acids involves a hydrogenation using any conventional catalyst and preferably Raney nickels and Raney cobalts.

13. The process as claimed in any of claims 1 to 12, **characterized in that** the treated charge is in the form of an ω-unsaturated fatty acid ester.
